(19) Europäisches Patentamt
European Patent Office
Office européen des brevets

(11) **EP 1 878 444 B1**

(12) **EUROPEAN PATENT SPECIFICATION**

(45) Date of publication and mention
of the grant of the patent:
**09.09.2015 Bulletin 2015/37**

(21) Application number: **06745880.2**

(22) Date of filing: **28.04.2006**

(51) Int Cl.:
*A61K 45/06* (2006.01)     *A61K 31/13* (2006.01)
*A61K 31/445* (2006.01)     *A61K 47/30* (2006.01)
*A61P 25/28* (2006.01)

(86) International application number:
**PCT/JP2006/309021**

(87) International publication number:
**WO 2006/118265 (09.11.2006 Gazette 2006/45)**

(54) **COMPOSITION CONTAINING ANTI-DEMENTIA DRUG**

ZUSAMMENSETZUNG MIT EINEM ARZNEIMITTEL GEGEN DEMENZ

COMPOSITION CONTENANT UN MÉDICAMENT ANTI-DÉMENCE

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **28.04.2005 JP 2005132593**

(43) Date of publication of application:
**16.01.2008 Bulletin 2008/03**

(73) Proprietor: **Eisai R&D Management Co., Ltd.
Tokyo 112-8088 (JP)**

(72) Inventors:
• **KIMURA, Susumu
Kakamigahara-shi,
Gifu 5016195 (JP)**
• **UEKI, Yosuke
Kakamigahara-shi,
Gifu 5016195 (JP)**
• **NOHARA, Masami
Kakamigahara-shi,
Gifu 5016195 (JP)**
• **DOTA, Yukifumi
Kakamigahara-shi,
Gifu 5016195 (JP)**

(74) Representative: **Hoffmann Eitle
Patent- und Rechtsanwälte PartmbB
Arabellastraße 30
81925 München (DE)**

(56) References cited:
| | |
|---|---|
| EP-A1- 1 832 298 | WO-A1-97/14415 |
| WO-A1-02/058676 | WO-A1-03/101458 |
| JP-A- 03 193 733 | JP-A- 09 188 617 |
| JP-A- 10 017 497 | JP-A- 2003 267 889 |
| JP-A- 2004 521 146 | US-A- 6 036 973 |
| US-A1- 2004 087 658 | US-B1- 6 262 081 |

## Description

Technical Field

The present invention relates to a composition comprising the two anti-dementia drugs donepezil or a pharmacologically acceptable salt thereof, and memantine or a pharmacologically acceptable salt thereof.

Background Art

In recent years, care for dementia such as senile dementia and Alzheimer-type dementia has become a social problem, and many therapeutic drugs for dementia are being developed. Of these, donepezil, which has been supplied as the hydrochloride in a tablet or granule form (trade name Aricept, manufactured by Eisai Co., Ltd.), is seen as being highly useful as a therapeutic drug for mild to moderate Alzheimer-type dementia due to having an acetylcholinesterase inhibiting action. Moreover, memantine hydrochloride, which exhibits antagonism towards N-methyl-D-aspartate (NMDA) receptors, has also been developed as a therapeutic drug for moderate to severe Alzheimer-type dementia, and has been supplied in a film-coated tablet or liquid form (trade name Axura, manufactured by Merz Pharmaceuticals; trade name Namenda, manufactured by Forest Pharmaceuticals, Inc.).

Recently, trials have been made using these two drugs together. It has been reported that upon further administering memantine hydrochloride or a placebo using a double blind test method to patients with moderate to severe Alzheimer-type dementia who had already been administered donepezil hydrochloride, for the group administered both donepezil hydrochloride and memantine hydrochloride, cognitive ability and activities of daily living were improved as compared to the group administered the placebo (see Non-Patent Document 1). Moreover, the idea of a preparation containing an acetylcholinesterase inhibitor and an NMDA receptor antagonist has also been disclosed (see Patent Document 1, and Patent Document 2).

Meanwhile, most Alzheimer-type dementia patients not only have reduced cognitive ability, but also have difficulty in swallowing, and so sufficient care must be taken with regard to compliance by the patients themselves, and also reducing the burden on care-givers. However, in the case of a therapeutic method in which commercially available products are used together, it is necessary, for example, to administer one donepezil hydrochloride tablet once per day, and further administer one memantine hydrochloride tablet twice per day; the frequency of administration and the amounts taken are thus high, and hence problems have arisen with regard to compliance. Moreover, in the case of a composition containing two or more kinds of drugs, the drugs have different solubilities and pKa values to one another. It is thus difficult to simultaneously control the release of two or more anti-dementia drugs in a single dosage form, and the current state of affairs is that specific control methods for anti-dementia drugs have not been disclosed in any publicly known literature, and furthermore there have also been no suggestions with regard to the need to improve compliance, or techniques for producing a preparation giving a combined effect of two or more anti-dementia drugs used together.

Patent Document 1 : International Publication No. 03/101458
Patent Document 2 : U.S. Patent Application Publication No. 2004/0087658
Non-Patent Document 1 : Pierre N. Tariot et al., "Memantine Treatment in Patients with Moderate to Severe Alzheimer Disease Already Receiving Donepezil - a Randomized Controlled Trial", JAMA, Vol. 291, No. 3, p. 317-324

Disclosure of Invention

Problems to be Solved by the Invention

As described above, for the case of implementing therapy in which at least two kinds of anti-dementia drugs are used together, there is a demand for a composition which has a good therapeutic effect on dementia, and which also gives excellent compliance. More specifically, there is a demand for a composition containing at least two kinds of anti-dementia drugs, for which release of the anti-dementia drugs from the composition is controlled, whereby a combined effect of the anti-dementia drugs can be achieved well. Furthermore, there is a demand for the development of a composition containing at least two kinds of anti-dementia drugs, according to which the frequency of administration and the amount taken are reduced, and hence compliance can be improved.

Moreover, from the standpoint of productivity and cost, there is a demand for the development of a composition which can be easily manufactured and which enables the release of at least two kinds of drugs to be easily controlled in accordance with the object.

Furthermore, there is a demand for a composition in which the blood concentration profiles for donepezil hydrochloride and memantine hydrochloride are equivalent to one another, or a pharmaceutical preparation in which, for each drug, there is an equivalent relationship between dissolution in an acidic solution and dissolution in a neutral solution so that the blood concentration profile for the drug is not affected by the gastric emptying time.

Means for Solving the Problems

In view of the above circumstances, the present inventors carried out assiduous studies in the quest for a composition which contains at least two kinds of anti-dementia drugs, and which is effective for dementia, and furthermore can be administered as infrequently as once per day, and hence gives excellent compliance. As a result, the present inventors have discovered that the desired objects can be attained through the following construction, thus arriving at the present invention.

In other words, the present invention provides a composition comprising donepezil or a pharmaceutically acceptable salt thereof, and memantine or a pharmacologically acceptable salt thereof, wherein the composition comprises at least one sustained-release portion containing comprising a non-pH-dependent polymeric substance wich comprises at least one selected from ethylcellulose, and an ethyl acrylate-methyl methacrylate copolymer, and a pH-dependent polymeric substance which comprises at least one selected from a methacrylic acid-ethyl acrylate copolymer, a methacrylic acid-methyl methacrylate copolymer, hydroxypropyl methylcellulose phthalate, and hydroxypropyl methylcellulose acetate succinate. The sustained-release portion further comprises at least one selected from donepezil or a pharmacologically acceptable salt thereof and memeantine or a pharmacologically acceptable salt thereof.

In a preferable aspect of the present invention, there is provided the above composition containing memantine hydrochloride in one sustained-release portion, and donepezil hydrochloride in another sustained-release portion.

In a preferable aspect of the invention, there is provided the above composition, wherein the non-pH-dependent polymeric substance is ethylcellulose.

In another preferable aspect of the invention, there is provided the above composition, wherein the pH-dependent polymeric substance is a methacrylic acid-ethyl acrylate copolymer.

In yet another preferable aspect of the present invention, there is provided the above composition wherein the sustained-release portion comprises granules or a compression-molded product.

In a preferable embodiment of the present invention, there is provided a composition in which dissolution of anti-dementia drugs can be controlled in accordance with the object. For example, according to the present invention, there is provided a composition in which dissolution of donepezil hydrochloride and memantine hydrochloride can be controlled. Specifically, the composition of the present invention can be specified by the dissolution profile or the change in dissolution percentage with dissolution time in an *in vitro* dissolution test, or the $f_2$ function value or the like. In this case, for such a composition, at least two kinds of drugs can be released from a carrier having the same composition.

According to the present invention, there can be provided, as a composition in which donepezil and memantine are made to be sustained-release, a composition specified by specific dissolution profiles in a neutral dissolution test solution. It can be made to be such that at least 80% of each of the two anti-dementia drugs is released in a specified dissolution time of 3 to 10 hours. In this case, the dissolution times for the anti-dementia drugs may be made to be the same as one another, or different to one another.

Further, according to the present invention, there can be provided a composition in which one of the two anti-dementia drugs is released at an early stage in an acidic region, and the other anti-dementia drug is released at a late stage in a neutral region. For example, there can be provided a composition in which at least 80% of one anti-dementia drug is released within a dissolution time of 3 hours, and at least 80% of the other anti-dementia drug is released in a specified dissolution time of 3 to 10 hours.

Furthermore, according to the present invention, there can be provided a composition in which donepezil and memantine are both released at an early stage in an acidic region. For example, there can be provided a composition in which the dissolution percentage for each of the two of anti-dementia drugs in an acidic dissolution test solution is at least 60% at a dissolution time of 1 hour.

Moreover, according to the present invention, there can be provided a composition in which the dissolution profiles for the two anti-dementia drugs are similar to or the same as one another. For example, the composition can be specified by a ratio of the dissolution percentages for the two kinds of anti-dementia drugs at certain dissolution times at which the dissolution percentages are compared, or the $f_2$ function value.

Furthermore, according to the present invention, there can be provided a composition in which the dissolution profile in an acidic dissolution test solution and the dissolution profile in a neutral dissolution test solution are closely similar or equivalent to one another for the two anti-dementia drugs. In this case, the similarity or equivalency of the dissolution profiles can be specified by a ratio of the dissolution percentage in the acidic dissolution test solution to the dissolution percentage in the neutral dissolution test solution, or the $f_2$ function value.

Advantageous Effect of the Invention

According to the composition of the present invention, not only can the effects of each of donepezil or a pharmaceutically acceptable salt thereof or memantine or a pharmaceutically acceptable salt thereof be achieved, but moreover there can be provided a novel therapeutic method due to a synergistic effect between these two anti-dementia drugs. In particular, according to the present invention, there can be provided a composition containing donepezil or a pharmaceutically acceptable salt thereof or memantine or a pharmaceutically acceptable salt thereof, in which dissolution can be controlled in accordance with the symptoms and state of the patient and the therapeutic method. Furthermore, according to the composition of the present invention, there can be provided a medicine that gives excellent compliance and is of excellent quality, and can be taken without anxiety by a patient exhibiting symptoms of dementia, or reduction in the burden on a care-giver administering the medicine can be realized. Furthermore, according to the present invention, design of a pharmaceutical preparation conforming to intended objectives with regard to controlling release of the two anti-dementia drugs can be carried out easily without using a special manufacturing apparatus, and moreover there can be provided a simple, convenient manufacturing method for a pharmaceutical composition in which the anti-dementia drugs are stabilized.

Brief Description of Drawings

FIG. 1 is a table showing component mixing proportions for components in examples of compositions according to the present invention;
FIG. 2 illustrates tables showing dissolution test evaluation results for Examples 1 and 2;
FIG. 3 illustrates a table showing dissolution test evaluation results for Examples 5 to 8;
FIG. 4 illustrates a table showing $f_2$ function values for dissolution profiles for two kinds of anti-dementia drugs; and
FIG. 5 illustrates a table showing $f_2$ function values for dissolution profiles in acidic and neutral dissolution test solutions.

Best Mode for Carrying Out the Invention

The following is a description of embodiments of the present invention. However, the following embodiments are merely illustrative for explaining the present invention, and it is not intended that the present invention be limited only to these embodiments. The present invention can be implemented in various modes, so long as there is no departure from the scope of the invention.

(Anti-dementia drugs)

The composition according to the present invention comprises donepezil or a pharmacologically acceptable salt thereof, and memantine or a pharmacologically acceptable salt thereof. Note that each donepezil and memantine may be used either in free form, or as an organic acid salt or inorganic acid salt, with an organic acid salt or inorganic acid salt being preferable, and an inorganic acid salt being particularly preferable.
Anti-dementia drugs preferably used in the present invention are tacrine, drugs, which may be a combination of anti-dementia drugs having the same
A combination of donepezil hydrochloride and memantine hydrochloride is Note that the composition according to the present invention may also contain therapeutic drugs other than anti-dementia drugs.

(Doses of drugs)

There are no particular limitations on the dose of each of donepezil and memantine for use in the composition of the present invention, but this dose is, for example, from 0.1 to 500 mg/day, preferably from 0.5 to 100 mg/day, more preferably from 1 to 50 mg/day.
In the case of donepezil or a pharmacologically acceptable salt thereof, the dose is preferably from 1 to 20 mg/day.
Moreover, in the case of memantine or a pharmacologically acceptable salt thereof, the dose is from 1 to 40 mg/day. The anti-dementia drug dose can be divided with the composition being administered a plurality of times per day, but the composition is preferably administered not more than once per day. The composition according to the present invention thus preferably contains at least one day's dose of each of the at least two kinds of the anti-dementia drugs as defined above.

(Control of release)

The composition according to the present invention enables controlled release from the composition containing the at least two kinds of anti-dementia drugs to be attained easily; for example, a method and form of administration in which administration is carried out once per day or less frequently than this can be realized. The term "controlled release" used in the present invention means that the release of the drugs from the composition is controlled in accordance with the object. When realizing such controlled release in the present invention, the release of the two of anti-dementia drugs can be controlled from a single preparation composition through either a sustained-release function or quick-. The term "sustained-release" herein not only indicates an anti-dementia drug being released more gradually over time than with quick-release, but also includes extended-release or pulsed-release in which release of the drug starts after a certain period of time, and prolonged release in which the drug concentration is maintained over time. dementia drug within 1 to 3 hours after commencement of dissolution in a

anti-dementia drug hitherto administered twice per day and an anti-dementia drug

hydrochloride and 10 mg of donepezil hydrochloride are made to be quick-release.

As yet another example, the two anti-dementia drugs memantine and donepezil that are usually used in different dose regimens can both be made to be sustained-release. For example, for a single composition, control can be carried out such that both 10 mg of donepezil hydrochloride and 20 mg of memantine hydrochloride are released gradually 6 to 12 hours after administration. Alternatively, the control of release of the two drugs can be carried out such that the donepezil hydrochloride is released gradually 6 to 12 hours after administration, and the memantine hydrochloride is subjected to pulsed-release immediately after administration and 6 to 8 hours after administration.

both controlled to be quick-release. An example is a composition containing 10 mg that is administered twice per day. Note that in the case of making the composition.

There are no particular limitations on each of the two anti-dementia drugs memantine and donepezil contained in the composition according to the present invention, but from the standpoint of controlling release, a basic drug or salt thereof that is less soluble in an alkaline aqueous solution than in an acidic aqueous solution, and for which the solubility with pH of an aqueous solution changes around a neutral pH is effective. Moreover, according to the composition of the present invention, control can be carried out simultaneously for an anti-dementia drug for which the change in solubility with pH of an aqueous solution around a neutral pH is relatively small, and an anti-dementia drug for which this change is relatively large. Each anti-dementia drug used in the present invention is, for example, a basic drug or a salt thereof for which the pKa of a basic functional group of the anti-dementia drug is from 7 to 12, preferably from 7.5 to 11, more preferably from 8 to 10.5, most preferably from 8.5 to 10.5. For example, donepezil hydrochloride is a basic drug with pKa = 8.90, and memantine hydrochloride is a basic drug with pKa = 10.27.

(Embodiment of composition)

The composition according to the present invention contains at least one sustained-release portion for performing a sustained-release function when controlling the release of the two anti-dementia drugs. Here, the term "containing at least one sustained-release portion" means that there may be one sustained-release portion, or a plurality of sustained-release portions, in the composition. A composition containing a sustained-release portion containing at least one anti-dementia drug is preferable. Each sustained-release portion in the present invention has a sustained-release function for one of the anti-dementia drugs. In this case, the form of the composition may be such that one sustained-release portion constitutes the whole composition, or may be such that the composition has at least one sustained-release portion as a part of the composition. Examples of the former include tablets or granules having a sustained-release film coating, and a matrix type sustained-release preparation having a wax or a resin as a base material. Moreover, the composition may be of a type in which a tablet containing sustained-release granules constituting a sustained-release portion is further coated with a sustained-release film so as to give the composition as a whole a sustained-release function. There is, however, no limitation to the above embodiments. Moreover, there are no particular limitations on the state of containment of each anti-dementia drug in the composition or in a quick-release portion or a sustained-release portion; the anti-dementia drug may be dispersed uniformly in the composition, or may be contained in only one part of the composition, or may be contained such that there is a concentration gradient.

Specific embodiments of the composition according to the present invention are given below, but there is no limitation thereto. Here, examples are given of various types of composition that can be administered once per day and contain, as the anti-dementia drugs, donepezil hydrochloride, which is usually administered once per day, and memantine hydrochloride, which is usually administered twice per day.

(Matrix type preparation)

A first example is a matrix type preparation. An aqueous solution of hydroxypropyl cellulose is added to a mixture of donepezil hydrochloride (manufactured by Eisai Co. Ltd.), memantine hydrochloride (manufactured by Lachema s.r.o., Czech Republic), ethylcellulose (Ethocel 10FP, manufactured by Dow Chemical Company, USA), Eudragit L100-55 (manufactured by Röhm GmbH & Co. KG, Darmstadt, Germany), and lactose, and wet granulation is carried out, and then the granules thus obtained are heat dried using a tray dryer, and then sieved to obtain the desired granule size. After sieving, magnesium stearate is added to the sustained-release granules obtained and mixing is carried out, and then a rotary tabletting machine is used to form a tablet, whereby a tablet containing 10 mg of donepezil hydrochloride and 20 mg of memantine hydrochloride can be obtained. Alternatively, it is also possible to prepare sustained-release granules for each of memantine hydrochloride and donepezil hydrochloride, then add sodium stearyl fumarate and carry out mixing, and then use a rotary tabletting machine to obtain a tablet. In this case, for each of the types of sustained-release granules, the amount of a non-pH-dependent polymeric substance or a pH-dependent polymeric substance according to the present invention can be varied in accordance with the release profiles of the two drugs. In any case, both donepezil hydrochloride and memantine hydrochloride can be made to be sustained-release, and hence such a tablet can be used as a tablet form administrable once per day.

(Gel matrix type preparation)

A second example is a gel matrix type preparation. Donepezil hydrochloride (manufactured by Eisai Co. Ltd.), memantine hydrochloride (manufactured by Lachema s.r.o., Czech Republic), and polyethylene oxide (Polyox, manufactured by Dow Chemical Company, USA), carboxyvinyl polymer (manufactured by BF Goodrich), and hydroxypropyl cellulose, each of these three polymers being water-swellable or forming a gel in water, are mixed together, and compression-molding is carried out using a rotary tabletting machine, whereby a compression-molded product can be obtained as a sustained-release portion. A film-coated tablet can then be obtained by using Opadry Yellow (Japan Colorcon) to further coat with a water-soluble film coating (coating amount: 5 mg/tablet) containing hydroxypropyl methylcellulose as a main component thereof. According to the resulting tablet, both donepezil hydrochloride and memantine hydrochloride can be made to be sustained-release, and hence the tablet can be used as a tablet form administrable once per day.

(Multi-layer tablet)

A third example is a tablet in which a plurality of layers are stacked on one another. In a two-layer tablet, the composition may be of a form administrable once per day in which the first layer is made to be a sustained-release portion containing 10 mg of donepezil hydrochloride and 10 mg of memantine hydrochloride, and the second layer is made to be a sustained-release portion from which 10 mg of memantine hydrochloride is released in a pulsed way. control of release being carried out freely in accordance with the kinds of the anti-

(Press-coated tablet)

A fourth example is a press-coated tablet having an inner core layer, and an outer layer covering the inner core layer. is a sustained-release portion. In this case, the inner core layer may contain hydrochloride is released in a pulsed way from an inner core layer. To make the

(Multi-granule preparation)

A fifth example is a composition containing a plurality of types of granules. Each of the types of granules can be made to be quick-release, sustained-release, pulsed-release or the like, so as to freely establish the desired dissolution profile. For example, gradually after administration. Alternatively, (2) a preparation form administrable once per day can be produced by making sustained-release granules for which release commences 2 hours, 4 hours, 6 hours, or 8 hours after administration contain 5 mg of memantine hydrochloride, and combining these with granules from which 10 mg of donepezil hydrochloride is released in a sustained way. There is no limitation to the above release profiles. Moreover, there are also no limitations on the dosage form of the preparation, which may be a granular preparation obtained by mixing the various types of granules together, or alternatively a tablet obtained by compression molding the various types of granules, or a capsule preparation obtained by filling the various types of granules into an HPMC capsule or the like.

(Multi-layered granules)

A sixth example is granules in which layers containing anti-dementia drugs are multi-layered on core particles of Nonpareil or the like. An example is granules in which a plurality of layers containing the anti-dementia drugs are multi-layered on Nonpareil 101 by alternately coating with a film coating liquid containing memantine hydrochloride and a film coating liquid containing donepezil hydrochloride. In this case, release of the anti-dementia drugs may be controlled by changing the concentration of the anti-dementia drug in each layer. Alternatively, sustained-release granules may be formed in which thin layers containing ethylcellulose and a plasticizer are provided between the layers containing the drugs and an outermost layer. Alternatively, a sustained-release function can be conferred to each of the layers containing the drugs by mixing the anti-dementia drug with ethylcellulose, Eudragit RS or the like in advance. Note that such granules can also be obtained by using granules containing at least one anti-dementia drug as the core particles instead of Nonpareil, and multi-layering layers containing the same anti-dementia drug or a different anti-dementia drug on these core particles. The resulting granules may be used alone, or a plurality of types of such granules may be combined; the granules may be used as the composition according to the present invention either as a granular preparation as is, or as a capsule preparation filled into HPMC capsules.

(Film-coated tablet)

A seventh example is a film-coated tablet. Memantine hydrochloride, donepezil hydrochloride, crystalline cellulose, lactose, and corn starch are mixed together, an aqueous solution of hydroxypropyl cellulose is added thereto, and wet granulation is carried out, and then the granules thus obtained are heat dried using a tray dryer, and then sieved to obtain the desired granule size. After sieving, magnesium stearate is added to granules obtained and mixing is carried out, and then a rotary tabletting machine is used to form a tablet, whereby a compression-molded product is obtained. further coat with a water-soluble film coating having hydroxypropyl methylcellulose Coating may be carried out with a mixture of a water-insoluble polymer such as ethylcellulose or Eudragit RS, and a water-soluble polymer or a plasticizer, so as to obtain a sustained-release film-coated tablet.

An eighth example is a composition in which a compression-molded product is taken as a quick-release portion, and sustained-release granules are dispersed in this compression-molded product. An example is a tablet obtained by mixing memantine hydrochloride and ethylcellulose together, and granulating to prepare sustained-release granules, and then mixing these sustained-release granules with donepezil hydrochloride, a diluent, a binder and so on, and compression-molding this mixture. The sustained-release granules may be granules having a single dissolution profile, or granules having a plurality of dissolution profiles as in the fifth example, or multi-layered granules as in the seventh example. Moreover, as the sustained-release portion, instead of sustained-release granules, a liposome or micro-capsules containing an anti-dementia drug may be contained.

(Dosage form)

There are no particular limitations on the dosage form of the composition according to the present invention, which may be any dosage form including tablets, capsules, granules, fine granules, a powder, orally rapid disintegrating tablets, an ointment, an injection, a poultice, a liquid, a preparation for per-tube administration, an inhalant, a jelly or the like. The dosage form is preferably one suitable for oral administration such as tablets, capsules, granules, fine granules, orally rapid disintegrating tablets, a liquid, a preparation for per-tube administration, or a jelly, with tablets, capsules, granules, fine granules, or orally rapid disintegrating tablets being particularly preferable.

(Additives for controlling release)

A sustained-release portion in the composition according to the present invention contains at least one non-pH-dependent polymeric substance or pH-dependent polymeric substance for controlling anti-dementia drug release, and preferably contains such a non-pH-dependent polymeric substance and such a pH-dependent polymeric substance.

(Non-pH-dependent polymeric substances)

The non-pH-dependent polymeric substance used in the present invention is a polymeric substance whose charge state hardly changes under pH conditions generally found in the gastrointestinal tract, specifically from pH 1 to pH 8. This means, for example, a polymeric substance that does not have functional groups whose charge state changes depending on the pH such as basic functional groups such as amino groups or acidic functional groups such as carboxylic acid groups. Note that in the present invention, the non-pH-dependent polymeric substance can be included for giving the composition according to the present invention a sustained-release function, but may also be included for another purpose. Moreover, the non-pH-dependent polymeric substance used in the present invention may be water-insoluble, or may swell in water or dissolve in water to form a gel. Examples of water-insoluble non-pH-dependent polymeric substances include, but are not limited to, cellulose ethers, cellulose esters, and methacrylic acid-acrylic acid copolymers (trade name Eudragit, manufactured by Röhm GmbH & Co. KG, Darmstadt, Germany). Examples include, but are not limited to, cellulose alkyl ethers such as ethylcellulose (trade name Ethocel, manufactured by Dow Chemical Company, USA), ethyl methylcellulose, ethyl propylcellulose or isopropylcellulose, and butylcellulose, cellulose aralkyl ethers such as benzyl cellulose, cellulose cyanoalkyl ethers such as cyanoethylcellulose, cellulose organic acid esters such as cellulose acetate butyrate, cellulose acetate, cellulose propionate or cellulose butyrate, and cellulose acetate propionate, ethyl acrylate-methyl methacrylate copolymers (trade name Eudragit NE, manufactured by Röhm GmbH & Co. KG, Darmstadt, Germany), and aminoalkyl methacrylate copolymer RS (trade names Eudragit RL, Eudragit RS). There are no particular limitations on the mean particle diameter of a water-insoluble polymer used in the present invention, but usually the lower this mean particle diameter the better the performance, with the mean particle diameter preferably being from 0.1 to 100 $\mu$m, more preferably from 1 to 50 $\mu$m, particularly preferably from 3 to 15 $\mu$m, most preferably from 5 to 15 $\mu$m. Moreover, examples of water-soluble or water-swelling non-pH-dependent polymeric substances include, but are not limited to, polyethylene oxide (trade name Polyox, manufactured by Dow Chemical Company, molecular weight 100,000 to 7,000,000), low-substituted hydroxypropyl cellulose (trade name L-HPC, manufactured by Shin-Etsu Chemical, Japan), hydroxypropyl cellulose (trade name HPC, manufactured by Nippon Soda, Co., Ltd, Japan), hydroxypropyl methylcellulose (trade names Metolose 60SH, 65SH, 90SH, manufactured by Shin-Etsu Chemical, Japan), and methylcellulose (trade name Metolose SM, manufactured by Shin-Etsu Chemical, Japan).

Note that in the present invention, at least one non-pH-dependent polymeric substance is contained in the composition. The composition may also comprise, or a plurality of the non-pH-dependent polymeric substances may . The non-pH-dependent polymeric substance used in the present invention is at least one selected from ethylcellulose, and an ethyl acrylate-methyl methacrylate copolymer (trade name Eudragit NE). Most preferable is ethylcellulose. There are no particular limitations on the amount of the non-pH-dependent polymeric substance contained in the composition; this amount can be adjusted as appropriate in accordance with the purpose such as controlling sustained drug release.


(pH-dependent polymeric substances)


The pH-dependent polymeric substance used in the present invention is a polymeric substance whose charge state changes under pH conditions generally found in the gastrointestinal tract, specifically from pH 1 to pH 8. This means, for example, a polymeric substance having functional groups whose charge state changes depending on the pH such as basic functional groups such as amino groups or acidic functional groups such as carboxylic acid groups. The pH-dependent functional groups of the pH-dependent polymeric substance are preferably acidic functional groups, with the pH-dependent polymeric substance most preferably having carboxylic acid groups.

The pH-dependent polymeric substance used in the present invention may be water-insoluble, or may swell in water or dissolve in water to form a gel. Examples of pH-dependent polymeric substances used in the present invention include, but are not limited to, enteric polymeric substances. Examples of enteric polymeric substances include, but are not limited to, methacrylic acid-methyl methacrylate copolymers (Eudragit L100, Eudragit S100, manufactured by Röhm GmbH & Co. KG, Darmstadt, Germany), methacrylic acid-ethyl acrylate copolymers (Eudragit L100-55, Eudragit L30D-55, manufactured by Röhm GmbH & Co. KG, Darmstadt, Germany), hydroxypropyl methylcellulose phthalate (HP-55, HP-50, manufactured by Shin-Etsu Chemical, Japan), hydroxypropyl methylcellulose acetate succinate (AQOAT, manufactured by Shin-Etsu Chemical, Japan), carboxymethyl ethylcellulose (CMEC, manufactured by Freund Corporation, Japan), and cellulose acetate phthalate. Examples of pH-dependent polymeric substances that swell in water or dissolve in water to form a gel include, but are not limited to, alginic acid, pectin, carboxyvinyl polymer, and carboxymethyl cellulose. In the present invention, a single pH-dependent polymeric substance may be contained in the composition, or a plurality of pH-dependent polymeric substances may be contained. The pH-dependent polymeric substance comprised in the composition of the present invention is at least one selected from a methacrylic acid-ethyl acrylate copolymer, a methacrylic acid-methyl methacrylate copolymer, hydroxypropyl methylcellulose phthalate, or hydroxypropyl methylcellulose acetate succinate, particularly preferably a methacrylic acid-ethyl acrylate copolymer.

When using a pH-dependent polymeric substance in the manufacturing process of the composition according to the present invention, a commercially available product of a powder type or a granular type, or a suspension type in which the pH-dependent polymeric substance has been dispersed in a solvent in advance can be used as is, or such a commercially available product can be used dispersed in water or an organic solvent. The lower the particle diameter of the pH-dependent polymeric substance the better the performance, with the pH-dependent polymeric substance preferably being of the powder type. In the case of a methacrylic acid-ethyl acrylate copolymer, an example is Eudragit L100-55. There are no particular limitations on the mean particle diameter of a pH-dependent polymeric substance used in the present invention, but the mean particle diameter is preferably from 0.05 to 100 $\mu$m, more preferably from 0.05 to 70 $\mu$m, most preferably from 0.05 to 50 $\mu$m. Moreover, there are no particular limitations on the amount of the pH-dependent polymeric substance, for example, in the case of an enteric polymeric substance, the amount is generally from 0.1 to 90 parts by weight, preferably from 1 to 70 parts by weight, more preferably from 5 to 60 parts by weight, particularly preferably from 10 to 50 parts by weight, based on 100 parts by weight of the composition.

(Additives)

The composition according to the present invention may further contain any of various additives, such as any of various pharmacologically acceptable carriers such as diluents, lubricants, binders and disintegrants, as well as preservatives, colorants, sweeteners, plasticizers, film coating agents and so on, as necessary. Examples of diluents include, but are not limited to, lactose, mannitol, dibasic calcium phosphate, starch, pregelatinized starch, crystalline cellulose, light silicic anhydride, synthetic aluminum silicate, magnesium aluminate metasilicate or the like. Examples of lubricants include, but are not limited to, magnesium stearate, calcium stearate, talc, sodium stearyl fumarate or the like. Examples of binders include, but are not limited to, hydroxypropyl cellulose, methylcellulose, sodium carboxymethyl cellulose, hydroxypropyl methylcellulose, polyvinylpyrrolidone or the like. Examples of disintegrants include, but are not limited to, carboxymethyl cellulose, calcium carboxymethyl cellulose, croscarmellose sodium, sodium carboxymethyl starch, low-substituted hydroxypropyl cellulose or the like. Examples of preservatives include, but are not limited to, paraoxybenzoic acid esters, chlorobutanol, benzyl alcohol, phenethyl alcohol, dehydroacetic acid, sorbic acid or the like. Preferable examples of colorants include, but are not limited to, water-insoluble lake pigments, natural pigments (e.g. β-carotene, chlorophyll, red ferric oxide), yellow ferric oxide, red ferric oxide, black ferric oxide or the like. Preferable examples of sweeteners include, but are not limited to, sodium saccharin, dipotassium glycyrrhizate, aspartame, stevia or the like. Examples of plasticizers include, but are not limited to, glycerol fatty acid esters, triethyl citrate, propylene glycol, polyethylene glycol or the like. Examples of film coating agents include, but are not limited to, hydroxypropyl methylcellulose, hydroxypropyl cellulose or the like.

(Manufacturing methods)

To manufacture the composition according to the present invention, a single conventional method, or a combination of conventional methods, can be used. For example, when manufacturing anti-dementia drug-containing granules as a sustained-release portion or a quick-release portion in the present invention, granulation is the main operation, but this may be combined with other operations such as mixing, drying, sieving, and classification. As the granulation method, for example, a wet granulation method in which a binder and a solvent are added to the powder and granulation is carried out, a dry granulation method in which the powder is compressed and granulation is carried out, a molten granulation method in which a binder that melts on heating is added and heating and granulation are carried out, or the like can be used. Furthermore, in accordance with the granulation method, an operating method such as a mixing granulation method using a planetary mixer, a screw mixer or the like, a high-speed mixing granulation method using a Henschel mixer, a Super mixer or the like, an extruding granulation method using a cylindrical granulator, a rotary granulator, a screw extruding granulator, a pellet mill type granulator or the like, a wet high-shear granulation method, a fluidized-bed granulation method, a compression granulation method, a crushing granulation method, or a spraying granulation method can be used. After the granulation, drying using a dryer, a fluidized bed or the like, cracking, and sieving can be carried out to obtain the granules or fine granules for use. Moreover, a granulation solvent may be used when preparing the composition according to the present invention. There are no particular limitations on such a granulation solvent, which may be water or any of various organic solvents, for example, water, a lower alcohol such as methanol or ethanol, a ketone such as acetone or methyl ethyl ketone, methylene chloride, or a mixture thereof.

(Method of manufacturing granules)

For sustained-release granules contained in the composition according to the present invention, at least one anti-dementia drug and at least one selected from non-pH-dependent polymeric substances and pH-dependent polymeric substances are mixed together, a diluent and a binder are added as necessary, and granulation is carried out to obtain granular matter. The granular matter obtained is dried using a tray dryer, a fluidized bed dryer or the like, and sieving is carried out using a mill or an oscillator, whereby the sustained-release granules can be obtained. Alternatively, as a method of manufacturing sustained-release granules in the present invention, it is possible to add at least one anti-dementia drug, at least one selected from non-pH-dependent polymeric substances and pH-dependent polymeric substances, and as necessary a diluent and a binder using a dry compactor such as a roller compactor or a slug tabletting machine, and carry out compression-molding while mixing, and then carry out granulation by cracking down to a suitable size. The granular matter prepared using such a granulator may be used as is as granules or fine granules according to the present invention, or may be further cracked using a power mill, a roll granulator, a rotor speed mill or the like, and sieved to obtain sustained-release granules. Note that quick-release granules can also be manufactured as for the sustained-release granules.

(Method of manufacturing compression-molded product)

A compression-molded product can be manufactured as an anti-dementia drug-containing sustained-release portion, or as the composition according to the present invention using a single conventional method, or a combination of conventional methods. For example, at least one anti-dementia drug, at least one selected from non-pH-dependent polymeric substances and pH-dependent polymeric substances, a diluent such as mannitol or lactose, a binder such as polyvinylpyrrolidone or crystalline cellulose, a disintegrant such as carmellose sodium or crospovidone, and a lubricant such as magnesium stearate or talc are used, and tableting is carried out using an ordinary method, whereby the compression molded product can be obtained. In this case, tabletting is the main operation in the method of manufacturing the compression-molded product, but this may be combined with other operations such as mixing, drying, sugar coating formation, and coating. Examples of the method for the tabletting include, but are not limited to, direct compression molding in which at least one anti-dementia drug and pharmacologically acceptable additives are mixed together and then the mixture is directly compression-molded into tablets using a tabletting machine, and dry granule compression or wet granule compression in which sustained-release granules or quick-release granules according to the present invention are subjected to compression-molding after adding a lubricant or a disintegrant as necessary. There are no particular limitations on the tabletting machine used in the compression molding; for example, a single-punch tabletting machine, a rotary tabletting machine, or a press-coated tabletting machine can be used.

(Coating method)

The anti-dementia drug-containing sustained-release granules, or compression-molded product according to the present invention can be used as is in the form of granules or a tablet as the composition of the present invention, but may also be subjected to further processing to manufacture the composition. For example, the compression-molded product or granules can be given a film coating using a film base material such as ethylcellulose, casein, methylcellulose, hydroxypropyl methylcellulose, methacrylic acid copolymer L, cellulose acetate phthalate, shellac or the like, or given a sugar coating using a sugar coating liquid containing saccharose, sugar alcohol, gum arabic powder, talc or the like, thus producing film-coated tablets or sugar-coated tablets. A preferable solvent in this coating technique is purified water, but an organic solvent such as an alcohol, a ketone, an ether or a chlorinated hydrocarbon, or a mixture thereof can also be used. For example, ethanol, acetone, methylene chloride or the like can be used as an organic solvent. Moreover, as the coating apparatus, an apparatus ordinarily used in coating techniques for manufacturing medicines can be used, with examples including a spray coating apparatus in which the coating is carried out by spraying a coating liquid or the like, and a rotor fluidized bed granulator for layering.

(Other manufacturing methods)

In the case of manufacturing capsule preparations, the capsule preparations can be manufactured by filling sustained-release granules, or mini-tablets into hard gelatin capsules or HPMC capsules using an automatic capsule filling machine. Alternatively, in the case of the preparations for per-tube administration or a dry syrup that is used mixed with water or the like when taken, sustained-release granules or quick-release granules as above can be mixed with a thickener or a dispersant so as to disperse these granules, the mixture then being made into granules or tablets. Furthermore, a liquid or jelly can be made using water, and substances selected from dispersants, emulsifiers, thickeners, preservatives, pH adjustors, sweeteners, flavorings, fragrances and so on. However, with respect to other manufacturing methods, there are no limitations to the above.

(Dissolution test)

With the composition of the present invention, release of the anti-dementia drugs can be controlled. A dissolution test method described in the Japanese Pharmacopoeia 14th Edition, USP or the like can be used for identifying a means of controlled release of the anti-dementia drugs, or for evaluating the state of controlled release. For example, measurement can be carried out using the first dissolution test method (rotating basket method), the second dissolution test method (paddle method), or the third dissolution test method (flow-through cell method) in the Japanese Pharmacopoeia. A composition with specified dissolution profile according to the present invention can be obtained using such a test method. For example, according to the present invention, a composition can be obtained in which, for each of two anti-dementia drugs contained in the composition, in a dissolution test, dissolution with little pH dependence can be secured during an early stage of dissolution, and then in a late stage of dissolution, proportion of the dissolution ratio in an acidic solution to the dissolution ratio in a neutral solution decreases over time as the dissolution test proceeds.
As a dissolution test solution, assuming gastric juice or intestinal juice, an aqueous solution of pH in a range of 1 to 9 can be used. For example, a buffer such as a phosphate buffer (e.g. a buffer prepared from a 50 mM sodium phosphate aqueous solution and hydrochloric acid), G.L. Miller buffer, Atkins-Pantin buffer, or Good buffer, or a 0.1 N hydrochloric acid solution, or a 0.1 mol/L sodium hydroxide solution, or the like can be used. The dissolution ratio is calculated by measuring the drug concentration at intervals of 15 minutes, 30 minutes, 1 hour, or 2 hours. Regarding the test period in the dissolution test, measurement is carried out until the dissolution ratio reaches at least 85%, or in the case of an acidic dissolution test solution, for at least 2 hours, or in the case of a neutral or basic dissolution test solution, for at least 24 hours.

(Controlled-release composition)

The composition according to the present invention contains at least the two anti-dementia drugs donepezil and memantine, with it being possible to control the dissolution of these anti-dementia drugs together or individually. For example, the present invention provides a composition, upon carrying out measurement using the second dissolution test method in the Japanese Pharmacopoeia with a paddle rate of 50 rpm, in which at least one anti-dementia drug contained in the composition has a dissolution ratio in a 0.1 N hydrochloric acid solution of pH 1 being from 20 to 50% at a dissolution time of 1 hour, and being from 85 to 100% at a dissolution time of 3 hours. Moreover, the composition may be such that, under the same dissolution conditions, at least one anti-dementia drug contained in the composition has a dissolution ratio in a 0.1 N hydrochloric acid aqueous solution of pH 1 being from 5 to 20% at a dissolution time of 1 hour, and being from 90 to 100% at a dissolution time of 8 hours. By combining such dissolution characteristics, the anti-dementia drugs contained in the composition according to the present invention can thus all be made to be sustained-release quick-release. Alternatively, one of the anti-dementia drugs can be made to be.

(Dissolution profiles)

According to the present invention, there can be provided, as a composition in which the two of anti-dementia drugs donepezil and memantine are made to be sustained-release, a composition specified by the dissolution profiles in a neutral (e.g. pH 6 to 8) dissolution test solution. Specifically, a composition in which, under the Japanese Pharmacopoeia paddle dissolution test method, the dissolution ratio for each of donepezil hydrochloride and memantine hydrochloride in a dissolution test solution of pH 6 to 8 is less than 60% at a dissolution time of 1 hour, and not less than 80% at a dissolution time of 8 hours. To delay the dissolution of these drugs, the dissolution ratio for each of donepezil hydrochloride and memantine hydrochloride is preferably less than 50%, more preferably less than 40%, at a dissolution time of 1 hour.

Moreover, according to the present invention, there can be provided a composition in which donepezil hydrochloride is released at an early stage in an acidic region (e.g. pH 1 to 3), and memantine hydrochloride is released at a late stage in a neutral region (e.g. pH 6 to 8). For example, there can be provided a composition in which, under the Japanese Pharmacopoeia paddle dissolution test method, the dissolution ratio for donepezil hydrochloride in a dissolution test solution of pH 1 to 2 is not less than 60% at a dissolution time of 1 hour, and the dissolution ratio for memantine hydrochloride in a dissolution test solution of pH 6 to 8 is less than 60% at a dissolution time of 1 hour and at not less than 80% at a dissolution time of 8 hours.

To accelerate the dissolution of donepezil hydrochloride, the dissolution ratio for donepezil hydrochloride is preferably not less than 80%, more preferably not less than 85%, at a dissolution time of 1 hour. Moreover, to delay the dissolution of memantine hydrochloride, the dissolution ratio for memantine hydrochloride is preferably less than 50%, more preferably less than 40%, at a dissolution time of 1 hour.

Furthermore, according to the present invention, there can be provided a composition in which memantine hydrochloride is released at an early stage in an acidic region, and donepezil hydrochloride is released at a late stage in a neutral region. For example, there can be provided a composition in which, under the Japanese Pharmacopoeia paddle dissolution test method, the dissolution ratio for memantine hydrochloride in a dissolution test solution of pH 1 to 2 is not less than 60% at a dissolution time of 1 hour, and the dissolution ratio for donepezil hydrochloride in a dissolution test solution of pH 6 to 8 is less than 60% at a dissolution time of 1 hour and not less than 80% at a dissolution time of 8 hours.

To accelerate the dissolution of memantine hydrochloride, the dissolution ratio for memantine hydrochloride is preferably not less than 80%, more preferably not less than 85%, at a dissolution time of 1 hour. Moreover, to delay the dissolution of donepezil hydrochloride, the dissolution ratio for donepezil hydrochloride is preferably less than 50%, more preferably less than 40%, at a dissolution time of 1 hour.

Furthermore, according to the present invention, there can be provided a composition in which both donepezil hydrochloride and memantine hydrochloride are released at an early stage in an acidic region. For example, there can be provided a composition in which, under the Japanese Pharmacopoeia paddle dissolution test method, the dissolution ratio for each of donepezil hydrochloride and memantine hydrochloride in a dissolution test solution of pH 1 to 2 is not less than 60%, preferably not less than 80%, more preferably not less than 85%, at a dissolution time of 1 hour.

(Proportion of dissolution ratios)

According to the present invention, there can be provided a composition in which dissolution profiles for donepezil hydrochloride and memantine hydrochloride are similar to or the same as one another. By making the dissolution characteristics be similar, a synergistic effect between the two drugs can be expected. For example, there can be provided a composition in which, for the dissolution ratios in the same dissolution test solution using the Japanese Pharmacopoeia paddle dissolution test method, the proportion of the dissolution ratio for memantine hydrochloride to the dissolution ratio for donepezil hydrochloride is in a range of $1 \pm 0.3$ at not less than three dissolution time points. Furthermore, to make the dissolution characteristics of the two anti-dementia drugs be similar to one another, this proportion of the dissolution ratios is preferably in a range of $1 \pm 0.2$, more preferably $1 \pm 0.15$.

Note that "the same dissolution test solution" refers to dissolution test solutions having the same composition and the same pH.

Moreover, the three or more dissolution time points at which the dissolution ratios are compared can be selected as desired. Specifically, in the case of assuming dissolution in the stomach and thus using an aqueous solution of pH 1 to 2 as the test solution, a plurality of dissolution time points between 15 minutes and 4 hours can be selected. Moreover, in the case of assuming dissolution in the intestines and thus using an aqueous solution of pH 6 to 8, a plurality of dissolution time points between 6 and 10 hours can be selected. For example, there can be provided a composition in which, under the Japanese Pharmacopoeia paddle dissolution test method using a dissolution test solution of pH 6 to 8, the proportion of the dissolution ratio for memantine hydrochloride to the dissolution ratio for donepezil hydrochloride is in a range of $1 \pm 0.3$ for each of the dissolution times 1 hour, 4 hours, and 8 hours.

Furthermore, there can be provided a composition in which the dissolution profiles at a late stage of dissolution are similar to or the same as one another. For example, there can be provided a composition in which, under the Japanese Pharmacopoeia paddle dissolution test method using a dissolution test solution of pH 6 to 8, the proportion of the dissolution ratio for memantine hydrochloride to the dissolution ratio for donepezil hydrochloride is in a range of $1 \pm 0.3$ for each of the dissolution times 6 hours, 8 hours, and 10 hours.

Furthermore, there can be provided a composition in which the dissolution profiles at an early stage of dissolution are similar to or the same as one another. For example, there can be provided a composition in which, under the Japanese Pharmacopoeia paddle dissolution test method using a dissolution test solution of pH 1 to 2, the proportion of the dissolution ratio for memantine hydrochloride to the dissolution ratio for donepezil hydrochloride is in a range of $1 \pm 0.3$ for each of the dissolution times 15 minutes, 30 minutes, and 45 minutes.

Moreover, there can be provided a composition in which the dissolution profiles are made to be similar to one another up to a dissolution time of 3 hours in an acidic dissolution test solution, and are made to be similar to one another over a dissolution time range of 4 to 8 hours in a neutral dissolution test solution.

As the dissolution time points at which the dissolution ratios are compared, dissolution times at which the dissolution ratio for one of donepezil or memantine reaches 30%, 50%, and 80% can be selected as desired. Specifically, there can be provided a composition in which, under the Japanese Pharmacopoeia paddle dissolution test method using a dissolution test solution of pH 6 to 8, the proportion of the dissolution ratio for memantine hydrochloride to the dissolution ratio for donepezil hydrochloride is in a range of $1 \pm 0.3$ for each of the dissolution times at which the dissolution ratio for one of the anti-dementia drugs is $30 \pm 5\%$, $50 \pm 5\%$, and $80 \pm 5\%$.

Moreover, as the dissolution time points at which the dissolution ratios are compared, the dissolution time at which the dissolution ratio reaches approximately 85% in the dissolution test can be selected together with 1/4, 1/2, and 3/4 of this dissolution time.

Specifically, there can be provided a composition in which, under the Japanese Pharmacopoeia paddle dissolution test method using a dissolution test solution of pH 6 to 8, the proportion of the dissolution ratio for memantine hydrochloride to the dissolution ratio for donepezil hydrochloride is in a range of $1 \pm 0.3$ for each of the dissolution time at which the dissolution ratio for donepezil hydrochloride reaches $85 \pm 5\%$, and 1/4, 1/2, and 3/4 of this dissolution time.

($f_2$ function)

The composition according to the present invention can be specified by a $f_2$ function value (similarity factor) calculated from the dissolution ratios in an *in vitro* dissolution test. For example, according to the present invention, there can be provided a composition in which, under the Japanese Pharmacopoeia paddle dissolution test method, the $f_2$ function value for the donepezil hydrochloride and memantine hydrochloride dissolution profiles is in a range of 42 to 100, i.e. a composition in which the dissolution profiles for the two kinds of anti-dementia drugs are similar to one another. The $f_2$ function value is preferably in a range of from 50 to 100, more preferably from 60 to 100. Note that, in general, the closer the $f_2$ function value is to 100, the more similar are the two dissolution profiles being compared, and if the $f_2$ function value is not less than 50, then it is considered that the two dissolution profiles being compared are "equivalent".

Here, the $f_2$ function is calculated using the following formula.

$$f_2 = 50 \log \left[ \frac{100}{\sqrt{1 + \dfrac{\sum\limits_{i=1}^{n} (Di-Ri)^2}{n}}} \right]$$

wherein Di and Ri are the dissolution ratios for the respective anti-dementia drugs, and n is the number of dissolution time points at which the dissolution ratios are compared.

Note that in the case that the dissolution ratio for one donepezil or memantine reaches not less than 85% in less than 30 minutes, the dissolution time points at which the dissolution ratios are compared are taken to be 15 minutes, 30 minutes, and 45 minutes (n = 3). Moreover, in the case that the dissolution time at which the dissolution ratio reaches not less than 85% is greater than 30 minutes for one of the anti-dementia drugs, taking a specified dissolution time at which the dissolution ratio reaches not less than 80% (hereinafter referred to as "T") as a standard time, the dissolution time points at which the dissolution ratios are compared are taken to be 1/4 T, 1/2 T, 3/4 T, and T (n = 4).

According to the present invention, there can also be provided a composition in which the dissolution in an acidic dissolution test solution and the dissolution in a neutral dissolution test solution are equivalent to one another for each of donepezil and memantine. According to such a composition, the risk of the blood concentration of the drugs varying upon the gastric emptying time changing can be reduced. For example, in the present invention, there can be provided a composition in which, under the Japanese Pharmacopoeia paddle dissolution test method, for dissolution times of 2 hours onwards, the proportion of the dissolution ratio in an acidic dissolution test solution to the dissolution ratio in a neutral dissolution test solution for donepezil hydrochloride is in a range of $1 \pm 0.3$, and the proportion of the dissolution ratio in an acidic dissolution test solution to the dissolution ratio in a neutral dissolution test solution for memantine hydrochloride is in a range of $1 \pm 0.3$. In this case, the proportion of the dissolution ratios is preferably in a range of $1 \pm 0.2$, more preferably $1 \pm 0.15$.

Specifying in terms of the $f_2$ function value, for example, there can be provided a composition in which, under the Japanese Pharmacopoeia paddle dissolution test method, the $f_2$ function value for the acidic dissolution test solution and neutral dissolution test solution dissolution profiles for donepezil hydrochloride is in a range of from 42 to 100, and moreover the $f_2$ function value for the acidic dissolution test solution and neutral dissolution test solution dissolution profiles for memantine hydrochloride is in a range of from 42 to 100. In this case, at least one of the $f_2$ function value for donepezil hydrochloride and the $f_2$ function value for memantine hydrochloride is preferably in a range of from 50 to 100, more preferably from 60 to 100.

Note that a dissolution test solution of pH 1 to 2 can be used as the acidic dissolution test solution, and a dissolution test solution of pH 6 to 8 can be used as the neutral dissolution test solution.

Techniques for making the release be sustained can be used, in particular, to solve problems of compliance for the patient taking donepezil and memantine. For example, the present invention provides a composition, upon carrying out measurement using the second dissolution test method in the Japanese Pharmacopoeia with a paddle rate of 50 rpm, in which donepezil and memantine contained in the composition has a proportion of the dissolution ratio for the anti-dementia drug in a 0.1 N hydrochloric solution, pH 1 to the dissolution ratio for the anti-dementia drug in a 50 mM phosphate buffer, pH 6.8 at a dissolution time of 3 hours of from 0.3 to 1.3. That is, the dissolution ratio while residing in the stomach is suppressed, or the speed of dissolution is made low, whereby the drug concentration in the blood plasma can be prevented from rising suddenly. The occurrence of side effects can thus be prevented, and there is a contribution to making the drug release sustained.

In another example, the present invention provides a composition, upon carrying out measurement using the second dissolution test method in the Japanese Pharmacopoeia with a paddle rate of 50 rpm, in which at least one anti-donepezil and memantine contained in the composition have a proportion of the dissolution ratio for the anti-dementia drug in a 0.1 N hydrochloric acid solution, pH 1 to the dissolution ratio for the anti-dementia drug in a 50 mM phosphate buffer, pH 6.8 that decreases with dissolution time up to the dissolution time at which the dissolution ratio in the 50 mM phosphate buffer, pH 6.8 is 90%. That is, the dissolution ratio in the stomach is kept low, and furthermore a decrease in the drug bioavailability as the composition passes from the stomach into the small intestine is inhibited, and hence the pharmacological effects can be achieved reliably.

With the composition according to the present invention, the release of a plurality of anti-dementia drugs can be controlled simultaneously in a single composition. For example, in the case of a composition containing two drugs having different solubilities to one another at pH 6.8, by producing a two-layer tablet formed from a first layer containing the less soluble anti-dementia drug and a second layer containing the more soluble anti-dementia drug, and making the total amount of non-pH-dependent polymeric substances and pH-dependent polymeric substances added as release-controlling substances be higher in the second layer than in the first layer, a desired sustained-release preparation can be obtained.

As another example, in the case of a composition containing two drugs having different solubility ratios between in a 0.1 N hydrochloric acid solution, pH 1 and in a 50 mM phosphate buffer, pH 6.8 (i.e. solubility in the 0.1 N hydrochloric acid solution, pH 1 / solubility in the 50 mM phosphate buffer, pH 6.8), by producing a two-layer tablet formed from a first layer containing the anti-dementia drug having the lower solubility ratio and a second layer containing the anti-dementia drug having the higher solubility ratio, and making the amount of pH-dependent polymeric substances based on 1 part by weight of non-pH-dependent polymeric substances be higher in the second layer than in the first layer, a desired sustained-release preparation can be obtained. Furthermore, as still another example, for two drugs having both (1) a different solubility in a 50 mM phosphate buffer, pH 6.8 and (2) a different solubility ratio between in a 0.1 N hydrochloric acid solution, pH 1 and in a 50 mM phosphate buffer, pH 6.8, by suitably adjusting both the total amount of non-pH-dependent polymeric substances and pH-dependent polymeric substances added as release-controlling substances, and the amount of the pH-dependent polymeric substances relative to the non-pH-dependent polymeric substances as in the above examples, a desired sustained-release preparation can be obtained.

In a preferable aspect of the present invention, there is provided a composition in which memantine hydrochloride and donepezil hydrochloride are contained in the same sustained-release portion. This is advantageous in terms of production efficiency and cost, since the at least two kinds of anti-dementia drugs can be controlled such as to have dissolution profiles as described above, and moreover both are in a single formulation. An example is a matrix type preparation containing donepezil hydrochloride and memantine hydrochloride as the anti-dementia drugs, containing non-pH-dependent polymeric substances and pH-dependent polymeric substances as release-controlling substances, and further containing pharmacologically acceptable additives. The matrix type preparation is preferably a tablet, a capsule preparation, granules, fine granules, or an orally rapid disintegrating tablet. Moreover, preferable release-controlling substances are ethylcellulose and a methacrylic acid-ethyl acrylate copolymer. Moreover, the matrix type preparation can be manufactured using a manufacturing method including a mixing step of mixing the anti-dementia drugs, the release-controlling substances and the pharmacologically acceptable additives together, and as necessary a granulation step of adding a binder to the mixture and granulating. In the case of a tablet or an orally rapid disintegrating tablet, this can be manufactured using a manufacturing method including a compression-molding step of compression molding the mixture obtained in the mixing step or the granular matter obtained in the granulation step. Furthermore, the manufacturing method may contain a step of coating the compression-molded product. Moreover, the granular matter obtained in the granulation step may be used as is as granules or fine granules, but the manufacture may also be carried out using a manufacturing method further including a step of mixing the granular matter with pharmacologically acceptable additives. A capsule preparation can be manufactured through a step of filling the granular matter obtained in the granulation step, or the granules or fine granules into a capsule.

In the present invention, regardless of whether the composition contains one sustained-release portion or a plurality of sustained-release portions, the content of release-controlling substances (non-pH-dependent polymeric substances and pH-dependent polymeric substances) in each sustained-release portion is generally from 1 to 99%, preferably from 5 to 90%, more preferably from 10 to 80%. Similarly, in the present invention, the content of the pH-dependent polymeric substances based on 1 part by weight of the non-pH-dependent polymeric substances in each sustained-release portion is generally from 0.1 to 20 parts by weight, preferably from 0.2 to 10 parts by weight, more preferably from 0.3 to 5 parts by weight.

The composition according to the present invention is, of course, not limited to the above. The composition according to the present invention is a composition in which dissolution control can be realized so as to achieve the effects of the anti-dementia drugs additively or synergistically, or so as to prevent or suppress the occurrence of side effects, or with some other objective, this being in accordance with the structural characteristics and physicochemical characteristics of the anti-dementia drugs.

Examples

The present invention is explained below in more detail with reference to the following examples, but the present invention should not be construed as being limited thereto. The additives used in the pharmaceutical compositions were reagents, or additives complying with official documents such as the Japanese Pharmacopoeia 14th Edition, Japanese Pharmaceutical Excipients 2003 (JPE), and the Japan Pharmaceutical Codex 1997 (JPC).

Example 1

6 g of donepezil hydrochloride (Eisai Co. Ltd.), 12 g of memantine hydrochloride (Lachema s.r.o.), 28.8 g of Ethocel 10FP (ethylcellulose, Dow Chemical Company), 36 g of Eudragit L100-55 (Röhm GmbH & Co. KG), and 45.6 g of lactose were mixed together in a granulator. An aqueous solution of 2.4 g of hydroxypropyl cellulose in a suitable amount of purified water was added to the mixture and wet granulation was carried out, and then the granules thus obtained were heat dried using a tray dryer, and then sieved to obtain the desired granule size. After sieving, 1 g of magnesium stearate based on 109 g of the granules was added and mixed in, and then a rotary tabletting machine was used to form tablets, whereby a compression-molded product with diameter 8 mm containing 10 mg of donepezil hydrochloride and 20 mg of memantine hydrochloride in a 220 mg tablet was obtained. Opadry yellow (Colorcon Japan Limited) was used to give the resulting product a water-soluble film coating containing hydroxypropyl methylcellulose as its main component (coating amount: 8 mg/tablet), resulting in film-coated tablets.

Reference Example 2

5 g of donepezil hydrochloride (Eisai Co. Ltd.), 10 g of memantine hydrochloride (Lachema s.r.o.), 20 g of corn starch (Nihon Shokuhin Kako Co., Ltd.), 15 g of crystalline cellulose (Asahi Kasei Corporation), and 81.75 g of lactose were mixed together in a granulator. An aqueous solution of 3.0 g of hydroxypropyl cellulose in a suitable amount of purified water was added to the mixture and wet granulation was carried out, and then the granules thus obtained were heat dried using a tray dryer, and then sieved to obtain the desired granule size. After the sizing, 0.25 g of magnesium stearate based on 134.75 g of the granules was added and mixed in, and then a rotary tabletting machine was used to form tablets, whereby a compression-molded product with diameter 7 mm containing 5 mg of donepezil hydrochloride and 10 mg of memantine hydrochloride in a 135 mg tablet was obtained. Opadry yellow (Colorcon Japan Limited) was used to give the resulting product a water-soluble film coating containing hydroxypropyl methylcellulose as its main component (coating amount: 5 mg / tablet), resulting in film-coated tablets.

Example 3

12 g of memantine hydrochloride (Lachema s.r.o.), 28.8 g of Ethocel 10FP (ethylcellulose, Dow Chemical Company), 36 g of Eudragit L100-55 (Röhm GmbH & Co. KG), and 39.6 g of lactose were mixed together in a granulator. An aqueous solution of 2.4 g of hydroxypropyl cellulose in a suitable amount of purified water was added to the mixture and wet granulation was carried out, and then the granules thus obtained were heat dried using a tray dryer, and then sieved to obtain the desired granule size. After sieving, 1 g of magnesium stearate based on 99 g of the granules was added and mixed in, and then a rotary tabletting machine was used to form tablets, whereby a compression-molded product with diameter 8 mm containing 20 mg of memantine hydrochloride in a 200 mg tablet was obtained. On the other hand, 3 g of donepezil hydrochloride (Eisai Co. Ltd.), 19.2 g of corn starch (Nihon Shokuhin Kako Co., Ltd.), 14.4 g of crystalline cellulose (Asahi Kasei Corporation), and 89.88 g of lactose were mixed together in a granulator. An aqueous solution of 2.88 g of hydroxypropyl cellulose in a suitable amount of purified water was added to the mixture and wet granulation was carried out, and then the granules thus obtained were heat dried using a tray dryer, and then sieved to obtain the desired granule size. After sieving, 0.4 g of magnesium stearate based on 215.6 g of the granules was added and mixed in, whereby a mixture containing donepezil hydrochloride was obtained. Subsequently, using 216 mg of the mixture containing donepezil hydrochloride per tablet of the compression-molded product containing memantine hydrochloride, a press-coated tabletting machine was used to form tablets, whereby press-coated tablets comprising an outer layer containing 5 mg of donepezil hydrochloride and an inner core layer containing 20 mg of memantine hydrochloride in a 416 mg tablet were obtained.

Example 4

6 g of donepezil hydrochloride (Eisai Co. Ltd.), 28.8 g of Ethocel 10FP (ethylcellulose, Dow Chemical Company), 36 g of Eudragit L100-55 (Röhm GmbH & Co. KG), and 57.6 g of lactose were mixed together in a granulator. An aqueous solution of 2.4 g of hydroxypropyl cellulose in a suitable amount of purified water was added to the mixture and wet granulation was carried out, and then the granules thus obtained were heat dried using a tray dryer, and then sieved to obtain the desired granule size. After sieving, 1 g of magnesium stearate based on 99 g of the granules was added and mixed in, and then a rotary tableting machine was used to form tablets, whereby a compression-molded product with diameter 8 mm containing 10 mg of donepezil hydrochloride in a 200 mg tablet was obtained. On the other hand, 6 g of memantine hydrochloride (Lachema s.r.o.), 19.2 g of corn starch (Nihon Shokuhin Kako Co., Ltd.), 14.4 g of crystalline cellulose (Asahi Kasei Corporation), and 86.88 g of lactose were mixed together in a granulator. An aqueous solution of 2.88 g of hydroxypropyl cellulose in a suitable amount of purified water was added to the mixture and wet granulation was carried out, and then the granules thus obtained were heat dried using a tray dryer, and then sieved to obtain the desired granule size. After sieving, 0.4 g of magnesium stearate based on 215.6 g of the granules was added and mixed in, whereby a mixture containing memantine hydrochloride was obtained. Subsequently, using 216 mg of the mixture containing memantine hydrochloride per tablet of the compression-molded product containing donepezil hydrochloride, a press-coated tableting machine was used to form tablets, whereby press-coated tablets comprising an outer layer containing 10 mg of memantine hydrochloride and an inner core layer containing 10 mg of donepezil hydrochloride in a 416 mg tablet were obtained.

Example 5

6 g of donepezil hydrochloride (Eisai Co. Ltd.), 12 g of memantine hydrochloride (Lachema s.r.o.), 30 g of Ethocel 10FP (ethylcellulose, Dow Chemical Company), 18 g of Eudragit L100-55 (Röhm GmbH & Co. KG), and 50.04 g of lactose (trade name Pharamatose 200M, DMV Japan) were mixed together in a granulator. An aqueous solution of 3.6 g of hydroxypropyl cellulose (trade name HPC-L, Nippon Soda, Co., Ltd, Japan) in a suitable amount of purified water was added to the mixture and wet granulation was carried out. The granules thus obtained were then heat dried using a tray dryer, and then sieved to obtain the desired granule size. After sieving, 0.36 g of magnesium stearate based on 119.64 g of the granules was added and mixed in, and then a single-punch tabletting machine was used to form tablets, whereby a compression-molded product with diameter 8 mm containing 10 mg of donepezil hydrochloride and 20 mg of memantine hydrochloride in a 200 mg tablet was obtained.

Examples 6 to 9

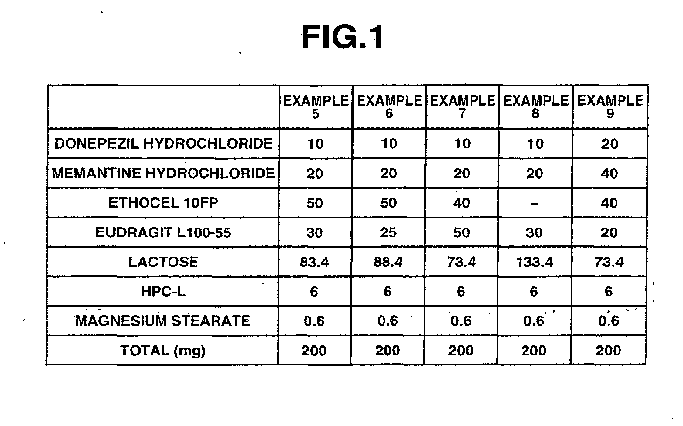

Compression-molded products were obtained using the same method as in Example 5. The mixing proportions of the components were as shown in FIG. 1.

(Dissolution tests)

Dissolution tests were carried out using the compression-molded products obtained in the examples. Each dissolution test was carried out in accordance with the dissolution test method described in the Japanese Pharmacopoeia 14th Edition, using test solution A indicated below as an acidic test solution, and test solution B indicated below as a neutral test solution, with a paddle rate of 50 rpm.
Test solution A: 0.1 N hydrochloric acid solution (exhibiting pH of 1 to 2)
Test solution B: 50 mM phosphate buffer of pH 6.8 (buffer of 50 mM sodium phosphate solution adjusted to pH 6.75 to 6.84 with hydrochloric acid)

<Measurement for donepezil hydrochloride>

For the donepezil hydrochloride dissolution ratio, the donepezil hydrochloride concentration in each of sample solutions collected over time was calculated using spectrophotometric method or HPLC analysis. The spectrophotometric method was carried out under measurement conditions of a measurement wavelength at 315 nm and a reference wavelength at 650 nm. On the other hand, the HPLC analysis was carried out under the following measurement conditions: measurement column: Capcell Pak UG120 C18 (Shiseido), mobile phase: 0.1 % formic acid / acetonitrile = 82/18 mixture, detection wavelength: 230 nm.

<Measurement for memantine hydrochloride>

The memantine hydrochloride dissolution ratio was determined by calculating the memantine hydrochloride concentration in each of sample solutions collected over time using HPLC analysis after memantine hydrochloride was fluorescent labeled by Fluorescamine.
The conditions for fluorescence labeling and HPLC analysis are typically as follows: After sample solutions (1 mL) collected over time was mixed with borate buffer (9 mL), pH 9.0 (USP), an acetone solution (5 mL) containing Fluorescamine (1.2 mg/mL) was added and stirred enough. Water (10 mL) was also added into the above solution and mixed to obtain a test sample. The test sample was analyzed by HPLC. HPLC analysis was performed under measurement conditions; measurement column: CAPCELL PAK UG120 C18 (Shiseido) or a equivalent column, column temperature: 40°C, mobile phase: borate buffer, pH 9.0 (USP) / acetonitrile = 60 / 40 mixture; and detection conditions: fluorescence detector (excitation wavelength / detection wavelength = 391 nm / 474nm).

(Evaluation of film-coated tablets)

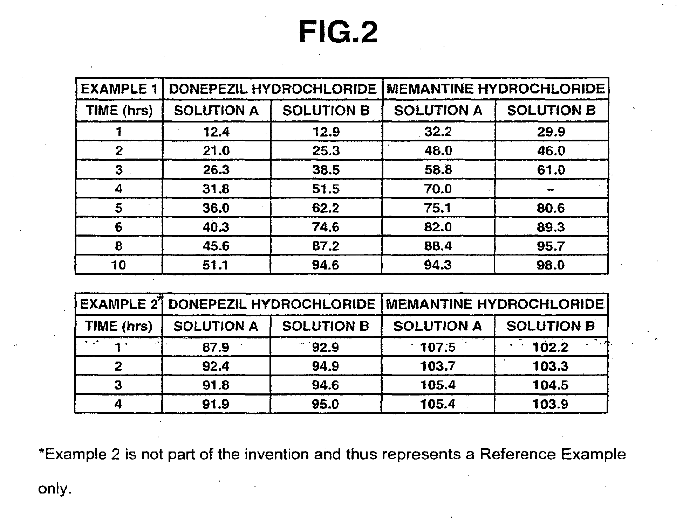

Evaluation results for the film-coated tablets of Example 1 and Reference Example 2 are shown in FIG. 2. For Example 1, donepezil hydrochloride and memantine hydrochloride both exhibited a sustained-release profile. For both drugs, the dissolution ratios in solution B was less than 30% at a dissolution time of 1 hour, and greater than 85% at a dissolution time of 8 hours. For Reference Example 2, the dissolution ratios for both donepezil hydrochloride and memantine hydrochloride showed quick-release. For both drugs, the dissolution ratio at a dissolution time of 1 hour was greater than 85%.

(Evaluation of compression-molded products)

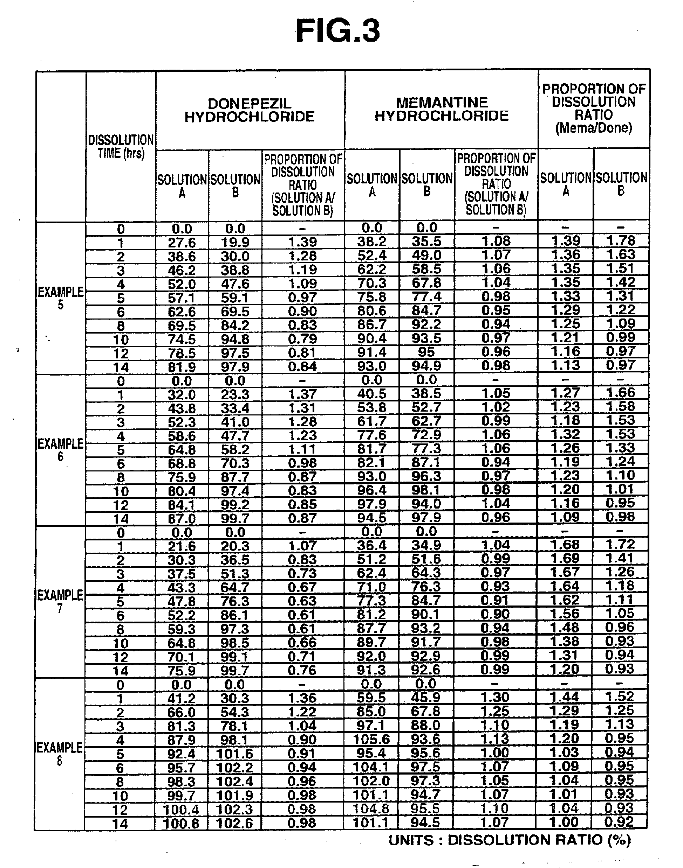

Dissolution tests were carried out using the compression-molded products of Examples 5 to 8. The results of dissolution ratios versus dissolution time are shown in FIG. 3. It was confirmed that by changing the content of ethylcellulose or Eudragit, compositions having various types of dissolution profiles for memantine hydrochloride and donepezil hydrochloride could be obtained.

(Similarity of dissolution profiles between drugs)

A proportion of the dissolution ratio for memantine hydrochloride to the dissolution ratio for donepezil hydrochloride (shown as "proportion of dissolution ratios (Mema / Done)" in table) is shown in FIG. 3. For example, for Example 6, it was confirmed that a composition was obtained in which the proportion of the dissolution ratios in solution A was in a range of $1 \pm 0.3$ at almost all dissolution times throughout the dissolution test, and the proportion of the dissolution ratios in solution B was $1 \pm 0.3$ in a late stage of dissolution from a dissolution time of 6 hours onwards. Moreover, for Example 7, the proportion of the dissolution ratios for donepezil hydrochloride and memantine hydrochloride in solution B was in a range of $1 \pm 0.3$ at dissolution times of 3 hours and beyond, showing that donepezil hydrochloride and memantine hydrochloride had similar dissolution profiles to one another.

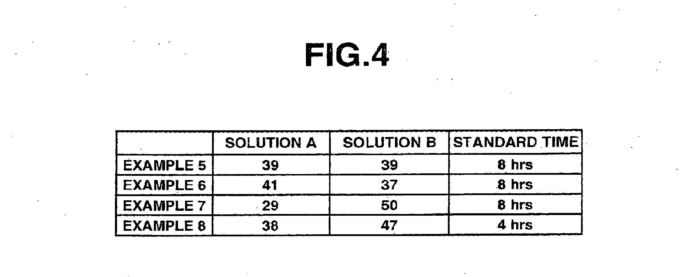

The $f_2$ function values for donepezil hydrochloride and memantine hydrochloride dissolution profiles were calculated based on the dissolution profile data of FIG. 3. Here, the standard time was taken to be a dissolution time of 8 hours or 4 hours. The results are shown in FIG. 4.

For Example 7, the $f_2$ function value for solution B was 50, showing that the donepezil hydrochloride and memantine hydrochloride dissolution profiles were equivalent. This preparation is a composition for which donepezil hydrochloride and memantine hydrochloride can be made to be sustained-release with the same dissolution profile in the same composition.

For Example 8, in the case of taking the standard time to be 4 hours, the $f_2$ function value for solution A was 38, and the $f_2$ function value for solution B was 47, and hence the dissolution profiles were found to be similar, showing that this preparation is useful for releasing the drugs within 4 hours after administration.

(Equivalency of dissolution profiles between dissolution test solutions)

The proportion of the dissolution ratio in solution A to the dissolution ratio in solution B for each of donepezil hydrochloride and memantine hydrochloride is shown in FIG. 3.

For Example 5, Example 6 and Example 8, the proportion of the dissolution ratios for donepezil hydrochloride and the proportion of the dissolution ratios for memantine hydrochloride were each in a range of $1 \pm 0.3$ at dissolution times of 2 hours onwards. In particular, for Examples 5 and 6, the proportion of the dissolution ratios for memantine hydrochloride was in a range of $1 \pm 0.1$ at dissolution times of 1 hour onwards.

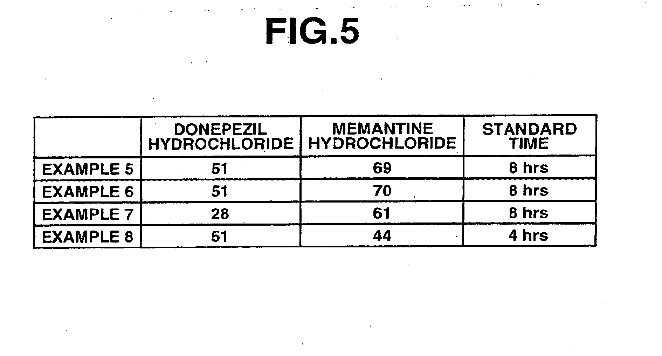

The $f_2$ function values for the dissolution profiles in solution A and solution B were calculated for each of donepezil hydrochloride and memantine hydrochloride based on the dissolution profile data of FIG. 3. Here, the standard time was taken to be a dissolution time of 8 hours or 4 hours. The results are shown in FIG. 5.

For Example 5 and Example 6, the $f_2$ function value was greater than 50 for each of the drugs, and hence it was found that the dissolution profiles were not affected much by the pH of the dissolution test solution. These compositions are useful as preparations not much affected by the gastric emptying time.

Industrial Applicability

According to the composition of the present invention, not only can the effects of donepezil and memantine be achieved, but moreover there can be provided a novel therapeutic method due to a synergistic effect between these two anti-dementia drugs. In particular, according to the present invention, there can be provided a composition containing donepezil and memantine in which dissolution is controlled in accordance with the symptoms and state of the patient and the therapeutic method. Furthermore, according to the composition of the present invention, there can be provided a medicine that gives excellent compliance and is of excellent quality, and can be taken without anxiety by a patient exhibiting symptoms of dementia, or in which the burden on a care-giver administering the medicine is reduced. Furthermore, according to the present invention, design of a preparation conforming to intended objectives with regard to controlling release of donepezil and memantine can be carried out easily without using a special manufacturing apparatus, and moreover there can be provided a simple, convenient manufacturing method for a pharmaceutical composition in which donepezil and memantine are stabilized.

**Claims**

1. A composition comprising:

   donepezil or a pharmacologically acceptable salt thereof; and
   memantine or a pharmacologically acceptable salt thereof;
   wherein the composition comprises a sustained-release portion comprising a non-pH-dependent polymeric substance which comprises at least one selected from ethylcellulose, and an ethyl acrylate-methyl methacrylate copolymer and a pH-dependent polymeric substance which comprises at least one selected from a methacrylic acid-ethyl acrylate copolymer, a methacrylic acid-methyl methacrylate copolymer, hydroxypropyl methylcellulose phthalate, and hydroxypropyl methylcellulose acetate succinate, and
   wherein the sustained-release portion comprises at least one selected from donepezil or a pharmacologically acceptable salt thereof and memantine or a pharmacologically acceptable salt thereof.

2. The composition according to Claim 1, wherein memantine hydrochloride is contained in one sustained-release portion and donepezil hydrochloride is contained in another sustained-release portion.

3. The composition according to Claims 1 to 2, wherein the non-pH-dependent polymeric substance is ethylcellulose.

4. The composition according to Claims 1 to 3, wherein the pH-dependent polymeric substance is a methacrylic acid-ethyl acrylate copolymer.

5. The composition according to any one of Claims 1 to 4, wherein the sustained-release portion comprises a granule or a compression-molded product.

**Patentansprüche**

1. Zusammensetzung, umfassend:

   Donepezil oder ein pharmakologisch annehmbares Salz davon; und
   Memantin oder ein pharmakologisch annehmbares Salz davon;
   wobei die Zusammensetzung einen Retard-Teil umfasst, der eine pH-unabhängige polymere Substanz, die zumindest eines, ausgewählt aus Ethylcellulose und einem Ethylacrylat-Methylmethacrylat-Copolymer, umfasst und eine pH-abhängige polymere Substanz, die zumindest eines, ausgewählt aus einem Methacrylsäure-Ethylacrylat-Copolymer, einem Methacrylsäure-Methylmethacrylat-Copolymer, Hydroxypropylmethylcellulosephthalat und Hydroxypropylmethylcelluloseacetatsuccinat, umfasst, umfasst und
   wobei der Retard-Teil zumindest eines, ausgewählt aus Donepezil oder einem pharmakologisch annehmbaren Salz davon und Memantin oder einem pharmakologisch annehmbaren Salz davon, umfasst.

2. Zusammensetzung gemäss Anspruch 1, wobei das Memantin-Hydrochlorid in einem Retard-Teil und das Donepezil-Hydrochlorid in einem anderen Retard-Teil enthalten ist.

3. Zusammensetzung gemäss Ansprüchen 1 bis 2, wobei die pH-unabhängige polymere Substanz Ethylcellulose ist.

4. Zusammensetzung gemäss Ansprüchen 1 bis 3, wobei die pH-abhängige polymere Substanz ein Methacrylsäure-Ethylacrylat-Copolymer ist.

5. Zusammensetzung gemäss irgendeinem der Ansprüche 1 bis 4, wobei der Retard-Teil ein Granulat oder ein druckgeformtes Produkt umfasst.

**Revendications**

1. Composition comprenant :

   du donépézil ou un sel pharmacologiquement acceptable de celui-ci ; et
   de la mémantine ou un sel pharmacologiquement acceptable de celle-ci ;
   dans laquelle la composition comprend une partie à libération prolongée comprenant une substance polymère indépendante du pH qui comprend au moins un élément choisi parmi de l'éthylcellulose, et un copolymère d'acrylate d'éthyle-méthacrylate de méthyle et une substance polymère dépendante du pH qui comprend au moins un élément choisi parmi un copolymère d'acide méthacrylique-acrylate d'éthyle, un copolymère d'acide méthacrylique-méthacrylate de méthyle, du phtalate d'hydroxypropylméthylcellulose, du succinate d'acétate d'hydroxypropylméthylcellulose, et
   dans laquelle la partie à libération prolongée comprend au moins un élément choisi parmi le donépézil ou un sel pharmacologiquement acceptable de celui-ci et la mémantine ou un sel pharmacologiquement acceptable de celle-ci.

2. Composition selon la revendication 1, dans laquelle du chlorhydrate de mémantine est contenu dans une partie à libération prolongée et du chlorhydrate de donépézil est contenu dans une autre partie à libération prolongée.

3. Composition selon les revendications 1 à 2, dans laquelle la substance polymère indépendante du pH est de l'éthylcellulose.

4. Composition selon les revendications 1 à 3, dans laquelle la substance polymère dépendante du pH est un copolymère d'acide méthacrylique-acrylate d'éthyle.

5. Composition selon l'une quelconque des revendications 1 à 4, dans laquelle la partie à libération prolongée comprend un granulé ou un produit moulé par compression.

# FIG.1

| | EXAMPLE 5 | EXAMPLE 6 | EXAMPLE 7 | EXAMPLE 8 | EXAMPLE 9 |
|---|---|---|---|---|---|
| DONEPEZIL HYDROCHLORIDE | 10 | 10 | 10 | 10 | 20 |
| MEMANTINE HYDROCHLORIDE | 20 | 20 | 20 | 20 | 40 |
| ETHOCEL 10FP | 50 | 50 | 40 | – | 40 |
| EUDRAGIT L100-55 | 30 | 25 | 50 | 30 | 20 |
| LACTOSE | 83.4 | 88.4 | 73.4 | 133.4 | 73.4 |
| HPC-L | 6 | 6 | 6 | 6 | 6 |
| MAGNESIUM STEARATE | 0.6 | 0.6 | 0.6 | 0.6 | 0.6 |
| TOTAL (mg) | 200 | 200 | 200 | 200 | 200 |

# FIG.2

| EXAMPLE 1 | DONEPEZIL HYDROCHLORIDE | | MEMANTINE HYDROCHLORIDE | |
|---|---|---|---|---|
| TIME (hrs) | SOLUTION A | SOLUTION B | SOLUTION A | SOLUTION B |
| 1 | 12.4 | 12.9 | 32.2 | 29.9 |
| 2 | 21.0 | 25.3 | 48.0 | 46.0 |
| 3 | 26.3 | 38.5 | 58.8 | 61.0 |
| 4 | 31.8 | 51.5 | 70.0 | – |
| 5 | 36.0 | 62.2 | 75.1 | 80.6 |
| 6 | 40.3 | 74.6 | 82.0 | 89.3 |
| 8 | 45.6 | 87.2 | 88.4 | 95.7 |
| 10 | 51.1 | 94.6 | 94.3 | 98.0 |

| EXAMPLE 2* | DONEPEZIL HYDROCHLORIDE | | MEMANTINE HYDROCHLORIDE | |
|---|---|---|---|---|
| TIME (hrs) | SOLUTION A | SOLUTION B | SOLUTION A | SOLUTION B |
| 1 | 87.9 | 92.9 | 107.5 | 102.2 |
| 2 | 92.4 | 94.9 | 103.7 | 103.3 |
| 3 | 91.8 | 94.6 | 105.4 | 104.5 |
| 4 | 91.9 | 95.0 | 105.4 | 103.9 |

*Example 2 is not part of the invention and thus represents a Reference Example only.

# FIG.3

| | DISSOLUTION TIME (hrs) | DONEPEZIL HYDROCHLORIDE | | | MEMANTINE HYDROCHLORIDE | | | PROPORTION OF DISSOLUTION RATIO (Mema/Done) | |
|---|---|---|---|---|---|---|---|---|---|
| | | SOLUTION A | SOLUTION B | PROPORTION OF DISSOLUTION RATIO (SOLUTION A/ SOLUTION B) | SOLUTION A | SOLUTION B | PROPORTION OF DISSOLUTION RATIO (SOLUTION A/ SOLUTION B) | SOLUTION A | SOLUTION B |
| EXAMPLE 5 | 0 | 0.0 | 0.0 | – | 0.0 | 0.0 | – | – | – |
| | 1 | 27.6 | 19.9 | 1.39 | 38.2 | 35.5 | 1.08 | 1.39 | 1.78 |
| | 2 | 38.6 | 30.0 | 1.28 | 52.4 | 49.0 | 1.07 | 1.36 | 1.63 |
| | 3 | 46.2 | 38.8 | 1.19 | 62.2 | 58.5 | 1.06 | 1.35 | 1.51 |
| | 4 | 52.0 | 47.6 | 1.09 | 70.3 | 67.8 | 1.04 | 1.35 | 1.42 |
| | 5 | 57.1 | 59.1 | 0.97 | 75.8 | 77.4 | 0.98 | 1.33 | 1.31 |
| | 6 | 62.6 | 69.5 | 0.90 | 80.6 | 84.7 | 0.95 | 1.29 | 1.22 |
| | 8 | 69.5 | 84.2 | 0.83 | 86.7 | 92.2 | 0.94 | 1.25 | 1.09 |
| | 10 | 74.5 | 94.8 | 0.79 | 90.4 | 93.5 | 0.97 | 1.21 | 0.99 |
| | 12 | 78.5 | 97.5 | 0.81 | 91.4 | 95 | 0.96 | 1.16 | 0.97 |
| | 14 | 81.9 | 97.9 | 0.84 | 93.0 | 94.9 | 0.98 | 1.13 | 0.97 |
| EXAMPLE 6 | 0 | 0.0 | 0.0 | – | 0.0 | 0.0 | – | – | – |
| | 1 | 32.0 | 23.3 | 1.37 | 40.5 | 38.5 | 1.05 | 1.27 | 1.66 |
| | 2 | 43.8 | 33.4 | 1.31 | 53.8 | 52.7 | 1.02 | 1.23 | 1.58 |
| | 3 | 52.3 | 41.0 | 1.28 | 61.7 | 62.7 | 0.99 | 1.18 | 1.53 |
| | 4 | 58.6 | 47.7 | 1.23 | 77.6 | 72.9 | 1.06 | 1.32 | 1.53 |
| | 5 | 64.8 | 58.2 | 1.11 | 81.7 | 77.3 | 1.06 | 1.26 | 1.33 |
| | 6 | 68.8 | 70.3 | 0.98 | 82.1 | 87.1 | 0.94 | 1.19 | 1.24 |
| | 8 | 75.9 | 87.7 | 0.87 | 93.0 | 96.3 | 0.97 | 1.23 | 1.10 |
| | 10 | 80.4 | 97.4 | 0.83 | 96.4 | 98.1 | 0.98 | 1.20 | 1.01 |
| | 12 | 84.1 | 99.2 | 0.85 | 97.9 | 94.0 | 1.04 | 1.16 | 0.95 |
| | 14 | 87.0 | 99.7 | 0.87 | 94.5 | 97.9 | 0.96 | 1.09 | 0.98 |
| EXAMPLE 7 | 0 | 0.0 | 0.0 | – | 0.0 | 0.0 | – | – | – |
| | 1 | 21.6 | 20.3 | 1.07 | 36.4 | 34.9 | 1.04 | 1.68 | 1.72 |
| | 2 | 30.3 | 36.5 | 0.83 | 51.2 | 51.6 | 0.99 | 1.69 | 1.41 |
| | 3 | 37.5 | 51.3 | 0.73 | 62.4 | 64.3 | 0.97 | 1.67 | 1.26 |
| | 4 | 43.3 | 64.7 | 0.67 | 71.0 | 76.3 | 0.93 | 1.64 | 1.18 |
| | 5 | 47.8 | 76.3 | 0.63 | 77.3 | 84.7 | 0.91 | 1.62 | 1.11 |
| | 6 | 52.2 | 86.1 | 0.61 | 81.2 | 90.1 | 0.90 | 1.56 | 1.05 |
| | 8 | 59.3 | 97.3 | 0.61 | 87.7 | 93.2 | 0.94 | 1.48 | 0.96 |
| | 10 | 64.8 | 98.5 | 0.66 | 89.7 | 91.7 | 0.98 | 1.38 | 0.93 |
| | 12 | 70.1 | 99.1 | 0.71 | 92.0 | 92.9 | 0.99 | 1.31 | 0.94 |
| | 14 | 75.9 | 99.7 | 0.76 | 91.3 | 92.6 | 0.99 | 1.20 | 0.93 |
| EXAMPLE 8 | 0 | 0.0 | 0.0 | – | 0.0 | 0.0 | – | – | – |
| | 1 | 41.2 | 30.3 | 1.36 | 59.5 | 45.9 | 1.30 | 1.44 | 1.52 |
| | 2 | 66.0 | 54.3 | 1.22 | 85.0 | 67.8 | 1.25 | 1.29 | 1.25 |
| | 3 | 81.3 | 78.1 | 1.04 | 97.1 | 88.0 | 1.10 | 1.19 | 1.13 |
| | 4 | 87.9 | 98.1 | 0.90 | 105.6 | 93.6 | 1.13 | 1.20 | 0.95 |
| | 5 | 92.4 | 101.6 | 0.91 | 95.4 | 95.6 | 1.00 | 1.03 | 0.94 |
| | 6 | 95.7 | 102.2 | 0.94 | 104.1 | 97.5 | 1.07 | 1.09 | 0.95 |
| | 8 | 98.3 | 102.4 | 0.96 | 102.0 | 97.3 | 1.05 | 1.04 | 0.95 |
| | 10 | 99.7 | 101.9 | 0.98 | 101.1 | 94.7 | 1.07 | 1.01 | 0.93 |
| | 12 | 100.4 | 102.3 | 0.98 | 104.8 | 95.5 | 1.10 | 1.04 | 0.93 |
| | 14 | 100.8 | 102.6 | 0.98 | 101.1 | 94.5 | 1.07 | 1.00 | 0.92 |

UNITS : DISSOLUTION RATIO (%)

EP 1 878 444 B1

EP 1 878 444 B1

# FIG.4

|  | SOLUTION A | SOLUTION B | STANDARD TIME |
|---|---|---|---|
| EXAMPLE 5 | 39 | 39 | 8 hrs |
| EXAMPLE 6 | 41 | 37 | 8 hrs |
| EXAMPLE 7 | 29 | 50 | 8 hrs |
| EXAMPLE 8 | 38 | 47 | 4 hrs |

23

# FIG.5

| | DONEPEZIL HYDROCHLORIDE | MEMANTINE HYDROCHLORIDE | STANDARD TIME |
|---|---|---|---|
| EXAMPLE 5 | 51 | 69 | 8 hrs |
| EXAMPLE 6 | 51 | 70 | 8 hrs |
| EXAMPLE 7 | 28 | 61 | 8 hrs |
| EXAMPLE 8 | 51 | 44 | 4 hrs |

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 03101458 A

- US 20040087658 A

**Non-patent literature cited in the description**

- **PIERRE N. TARIOT et al.** Memantine Treatment in Patients with Moderate to Severe Alzheimer Disease Already Receiving Donepezil - a Randomized Controlled Trial. *JAMA,* vol. 291 (3), 317-324